# EUROPÄISCHE PATENTSCHRIFT

(11) **EP 2 523 692 B1**
(45) Veröffentlichungstag und Bekanntmachung des Hinweises auf die Patenterteilung: **15.06.2016**
(21) Anmeldenummer: 11707562.2
(22) Anmeldetag: 11.01.2011
(51) Int. Cl.: A61K 47/48, A61K 45/06, A61P 35/00, A61P 31/00, A61P 33/00, C12N 15/11, A61K 31/7088

(54) **BIOLOGISCH WIRKSAME MOLEKÜLE ZUR BEEINFLUSSUNG VON VIRUS-, BAKTERIEN-, PARASITEN-INFIZIERTEN ZELLEN UND/ODER TUMORZELLEN UND VERFAHREN ZU DEREN ANWENDUNG**
BIOLOGICALLY ACTIVE MOLECULES FOR INFLUENCING VIRUS-, BACTERIA-, PARASITE-INFECTED CELLS AND/OR TUMOR CELLS AND METHOD FOR THE USE THEREOF
MOLÉCULES BIOLOGIQUEMENT ACTIVES, DESTINÉES À INFLUENCER DES CELLULES INFECTÉES PAR UN VIRUS, DES BACTÉRIES OU DES PARASITES ET/OU DES CELLULES TUMORALES ET PROCÉDÉ D'UTILISATION DE CES MOLÉCULES

(30) Priorität: 14.01.2010 DE 102010004957
(43) Veröffentlichungstag der Anmeldung: 21.11.2012
(73) Patentinhaber: Friedrich-Schiller-Universität Jena, 07743 Jena (DE)
(72) Erfinder: PÖHLMANN, Tobias, 08058 Zwickau (DE); GÜNTHER, Rolf, 22559 Hamburg (DE)
(74) Vertreter: Vossius & Partner Patentanwälte Rechtsanwälte mbB
(86) Internationale Anmeldenummer: PCT/DE2011/000024
(87) Internationale Veröffentlichungsnummer: WO 2011/085720

(56) Entgegenhaltungen:
- WO-A2-2008/098569

## Beschreibung

Die Erfindung betrifft biologisch wirksame Moleküle zur Beeinflussung von Virus-, Bakterien-, Parasiten-infizierten Zellen und/oder Tumorzellen sowie ein Verfahren zu deren Anwendung. Mit der Erfindung sollen die Virus-, Bakterien-, Parasiten-infizierten Zellen und/oder Tumorzellen mit einer oder mehreren Zielproteasen selbst für den Fall von Mutationen dieser Zielproteasen in ihrer Physiologie beeinflusst werden können.

Die vorgeschlagenen biologisch wirksamen Moleküle und deren spezielle Anwendung können insbesondere zur Bekämpfung und Wachstumshemmung anormaler Zellen, beispielsweise bei der Tumorbehandlung sowie bei der Behandlung von Virus-Infektionen, bakteriellen Infektionen oder bei Infektionen mit Parasiten eingesetzt werden.

Die Verwendung von Proteaseinhibitoren in der Bekämpfung von Virusinfektionen wurde bereits beschrieben und seit vielen Jahren verwendet (beispielsweise EP 0691345 A3; US 5,196,438 A; US 5,541,206 A; US 5,413,999 A; US 5,484,926 A; US 5,585,397 A).

Die Hemmung der Genexpression durch Einbringen von kurzen (19-23bp), doppelsträngigen RNA-Molekülen (siRNA) bzw. PNA-Molekülen in eukaryotische Zellen, die spezifisch für einen Sequenzabschnitt der mRNA eines Zielgens ist, wurde ebenfalls bereits beschrieben (Elbashir SM et al.: Duplexes of 21-nucleotide RNAs mediate RNA interference in cultured mammalian cells, Nature, 2001 May 24, 411(6836), 494-8; Liu Y et al.: Efficient and isoform-selective inhibition of cellular gene expression by peptide nucleic acids, Biochemistry, 2004 Feb 24, 43(7), 1921-7; US 5,898,031 A; US 7,056,704 B2).

Mit Hilfe solcher Moleküle wird nicht das Ablesen eines Gens und die Produktion einer mRNA verhindert, sondern es wird im Falle von siRNA ein zelleigener Mechanismus initiiert, der die Target-mRNA abbaut. Schließlich wird, wie vorbeschrieben, die Bildung eines spezifischen Proteins unterdrückt, ohne die Expression weiterer Gene zu beeinträchtigen (post-transcriptional gene silencing).
Um die Expression eines Gens zu unterdrücken, können die siRNA und PNA Moleküle dabei insbesondere über Transfektionsreagenzien und Elektroporation direkt in die Zelle eingebracht werden (Zhang M et al.: Downregulation enhanced green fluorescence protein gene expression by RNA interference in mammalian cells, RNA Biol. 2004 May, 1(1), 74-7; Gilmore IR et al.: Delivery strategies for siRNA-mediated gene silencing, Epub 2004 May 22., Curr Drug Deliv. 2006 Apr, 3(2), 147-5; US 6,506,559 B1).
Von Nachteil ist dabei, dass die siRNA relativ instabil ist, was durch chemische Modifikationen verbessert werden kann (US 6,107,094 A).

Problematisch bei der Anwendung der biologisch wirksamen Moleküle ist insbesondere eine Applikation in vivo. Für eine solche Applikation wurden Möglichkeiten entwickelt, beispielsweise siRNA Moleküle zu stabilisieren, um den Abbau zu vermindern (Morrissey et. al.: "Chemical Modifications of Synthetic siRNA", Pharmaceutical Discovery, May 1, 2005), und es wurden Transfektionsreagenzen, beispielsweise Nanopartikel, in vivo-jetPEI™, entwickelt, welche auch in vivo die siRNA in Zellen einbringen (Vernejoul et al.: Antitumor effect of in vivo somatostatin receptor subtype 2 gene transfer in primary and metastatic pancreatic cancer models, Cancer Research 62, 2002, 6124-31; Urban-Klein B, Werth S, Abuharbeid S, Czubayko F, Aigner A: RNAimediated gene-targeting through systemic application of polyethylenimine (PEI)-complexed siRNA in vivo, Gene Ther 12(5), 2005, 461-6.).

Ebenfalls wurden Methoden entwickelt, verstärkt Zellen eines Zielgewebes mit siRNA in vivo zu transfizieren (Ikeda et. al.: "Ligand-Targeted Delivery of Therapeutic siRNA", Pharmaceutical Research, Vol. 23, No. 8, August 2006).

Die Verabreichung von biologisch aktiven Substanzen in vivo ist allerdings auf Grund der systemischen Wirkung oft problematisch. Das Einbringen dieser Substanzen selektiv in Zielzellen erfolgt nicht ausreichend spezifisch. Dies ist besonders bei siRNA-, PNA- und RNA-Molekülen von Nachteil, die selektiv und ausschließlich in Zielzellen zur Wirkung kommen sollen. Durch gewebs- bzw. zellspezifisch-markierte Transfektionsreagenzien (z. B. Antikörper/Antigen-markierte Nanopartikel, TAT-Protein-Flankierung, u. a.) wird keine genügend große Zellspezifität erreicht. Fehltransfektionen sind die Folge.

Um die besagten Fehltransfektionen zu kompensieren, ist ein Mechanismus bekannt, mit dem siRNA-, PNA- oder RNA-Moleküle durch Bindung von Peptiden in ihrer biologischen Wirkung inhibiert und diese Peptide zum Zwecke der Aktivierung der siRNA, PNA oder RNA in den Zielzellen durch Zielzell-aktive Enzyme abgespalten werden (WO 2008098569 A2).

Ein generelles Problem der Anwendung von Proteaseinhibitoren zur Hemmung der Infektiösität oder Replikation von Viren, Bakterien oder Parasiten und des Wachstums von Tumoren ist, dass sich diese viralen, bakteriellen, parasitären oder Tumor-spezifischen Enzyme, beispielsweise durch Mutationen, sehr schnell geringfügig verändern und somit die verabreichten Inhibitoren nicht mehr wirken. Dadurch können sich die Viren, Bakterien, Parasiten oder die Tumorzellen trotz verabreichter Inhibitoren dennoch wieder vermehren.
Auch der Einsatz von siRNA-, RNA- oder PNA-Molekülen an sich wird auf Grund der schnellen Mutationsrate des Erbgutes in Virus-, Bakterien- oder Parasiten-infizierten Zellen oder Tumorzellen in der Praxis als nicht zweckmäßig bzw. nicht ausreichend wirkungsvoll angesehen, da auch Veränderungen der mRNA Zielsequenz der siRNA, PNA oder RNA den beabsichtigten Anwendungseffekt der eingesetzten Moleküle verhindern können.

Der Erfindung liegt die Aufgabe zu Grunde, gezielt Virus-, Bakterien-, Parasiten-infizierte Zellen und Tumorzellen selbst für den Fall von Mutationen bzw. Veränderungen der Zielprotease wirksam zu beeinflussen.

Erfindungsgemäß werden zur Beeinflussung der Virus-, Bakterien-, Parasiten-infizierten Zellen und/oder Tumorzellen biologisch wirksame Moleküle verabreicht, die sowohl aus zumindest einem Proteaseinhibitor für wenigstens eine spezifische Zielprotease der Virus-, Bakterien-Parasiten-infizierten Zellen und/oder Tumorzellen bestehen als auch aus zumindest einer Peptid-inhibierten siRNA, PNA oder RNA, deren Peptidbindung zum Zweck der Aktivierung der Peptid-inhibierten siRNA, PNA oder RNA durch die wenigstens eine spezifische Zielprotease aufgebrochen wird.

Der Proteaseinhibitor bindet in an sich bekannter Weise an die bestimmungsgemäße Zielprotease der Virus-, Bakterien-, Parasiten-infizierten Zellen bzw. Tumorzellen zum Zweck der Unterdrückung der biologischen bzw. Tumor-Aktivität der Zelle an. Für den Fall, dass der verabreichte Proteaseinhibitor, beispielsweise infolge einer geringfügigen Mutation der Zielprotease, nicht an diese binden und selbige in ihrer vorgenannten Wirkung nicht bestimmungsgemäß hemmen kann, (oder bei verbleibender Restaktivität der Zielprotease) wirkt die Zielprotease auf die gleichzeitig oder wenig zeitlich versetzt zum Proteaseinhibitor verabreichte Peptid-inhibierte siRNA, PNA oder RNA, deren Peptidbindung eine gleiche und/oder leicht veränderte Proteinsequenz der Aufbruchstelle der Zielprotease zum Zweck des Aufbruchs der Peptidbindung durch die Zielprotease aufweist. Mit dem Aufbrechen dieser Peptidbindung wird die Wirkung der siRNA, PNA oder RNA aktiviert, die nun ihrereseits auf die Physiologie der Zelle wirkt und die spezifische Expression des Zielgens vermindert. Beispielsweise wird dadurch die Expression eines für die Zelle überlebenswichtiges Gen ausgeschaltet und die Zelle wird dadurch abgetötet. Dabei interagieren die RNA-, siRNA- oder PNA-Moleküle nach ihrer Aktivierung mit der mRNA der Zielprotease und im Falle von siRNA bilden sie zusammen mit speziellen Endoribonukleasen einen RNA-Proteinkomplex mit der Bezeichnung "RISC" (RNA induced silencing complex). Der RISC Komplex bindet an die Target-mRNA, wobei Endonukleasen die Ziel-mRNA schneiden. Auf diese Weise wird in an sich bekannter Weise die Genexpression verhindert und somit das Entstehen von Zielproteinen gehemmt. Im Falle der Verwendung von aktivierten PNA-Molekülen wird mit der Bindung an die Ziel-mRNA die Translation verhindert.

Damit wird erreicht, dass beispielsweise im Falle von Virusinfektionen entweder der verabreichte Proteaseinhibitor an die spezifische Zielprotease bindet, diese wirksam inhibiert und dadurch eine Replikation des Virus verhindert wird, oder sollte durch die besagte (wenngleich auch nur geringfügige Veränderung) der Zielprotease eine Anbindung des Proteaseinhibitors an die Zielprotease nicht (oder nicht mehr) möglich sein (damit bliebe oder wäre wieder die Zielprotease aktiv, so dass eine Replikation des Virus möglich ist), dann aktiviert genau diese Zielprotease (welche durch den Proteaseinhibitor nicht gehemmt werden konnte) die besagte Peptid-inhibierte siRNA, PNA oder RNA, wodurch die Virus-infizierte Zelle beispielsweise abgetötet wird. Gleiches tritt ein, wenn der Proteaseinhibitor zwar an die Zielprotease anbindet, aber diese in ihrer Wirkung nicht vollends hemmt, so dass eine gewisse Restaktivität der Zielprotease verbleibt, durch welche ebenfalls eine Virusreplikation nicht auszuschließen wäre.

Für eine beispielhaft angeführte Behandlung von HIV bedeutet dies, dass die Zielprotease des HI-Virus entweder direkt durch einen Proteasehemmer inhibiert wird und somit zu einer verminderten Replikation führt oder (erfahrungsgemäß werden daraufhin HI-Virus-Mutanten selektiert, deren Protease durch den Proteasehemmer nicht oder weniger inhibiert wird) es wird dann der komplementäre Wirkmechanismus ausgehend von der genannten siRNA, PNA oder RNA aktiviert.
Auf diese Weise könnte beispielsweise die Expression von, für die Zelle überlebenswichtigen Genen vermindert und Apoptose- oder Nekroseprozesse ausgelöst werden.

Als Proteaseinhibitoren können beispielsweise an sich bekannte kleine Moleküle, Peptide, Proteine, insbesondere Antikörper, oder chemische Modifikationen davon Verwendung finden.

Der zumindest eine Proteaseinhibitor und die zumindest eine Peptid-inhibierte siRNA, PNA oder RNA können vorteilhaft gleichzeitig in einem gemeinsamen Molekül verabreicht werden, in welchem beide kovalent miteinander gebunden sind.

Allerdings ist es auch möglich, den zumindest einen Proteaseinhibitor und die zumindest eine Peptid-inhibierte siRNA in getrennten und nicht kovalent miteinander gebundenen Komplexen gleichzeitig oder wenig zeitlich zueinander versetzt zu applizieren.

Die Applikation der erfindungsgemäßen biologisch wirksamen Moleküle kann einmalig oder mehrmalig erfolgen, wobei im letztgenannten Fall der zumindest eine Proteaseinhibitor und die zumindest eine Peptid-inhibierte siRNA, PNA oder RNA bei weiterer Verabreichung mit ggf. durch Konzentration und/oder Molekülstruktur veränderter Wirkung auf die Virus-, Bakterien-, Parasiten-infizierten Zellen und/oder Tumorzellen eingesetzt werden.

Die in ihrer Wirkung zu hemmende Zielprotease kann je nach Anwendung eine virale Protease oder eine Protease eines Parasiten bzw. eines Bakteriums sein. Das Peptid zur Inhibition der siRNA, RNA oder PNA charakterisiert die natürliche oder abgewandelte Form der Schnittstelle der Protease als Bruchstelle, die von der Protease erkannt und dann aufgebrochen wird, inbesondere eine Schnittstelle, die auch von einer mutierten Form der Protease erkannt und geschnitten wird.

Vorteilhaft ist bei der Applikation der biologisch wirksamen Moleküle auch, wenn diese zusammen mit einem Transfektionsreagenz, insbesondere Lipide, Polyethylenimine, Nanopartikel, Polymere, Dextran verabreicht werden.

Gemeinsam mit der Applikation des zumindest einen Proteaseinhibitors und der zumindest einen Peptid-inhibierten siRNA, PNA oder RNA können weitere Wirkstoffe verabreicht werden, die ebenfalls die zellulären Eigenschaften der Virus-, Bakterien-, Parasiten-infizierten Zellen und/oder Tumorzellen oder die Replikation, Infektiösität, Verkapselung oder Freisetzung des Virus, Replikation, Infektiösität, Stoffwechsel oder Freisetzung eines Bakteriums oder eines Parasiten beeinflussen.

Die Peptid-inhibierte siRNA, RNA oder PNA kann wenigstens eine Anbindung weiterer Strukturen oder Funktionselemente besitzen, insbesondere für Rezeptor-Ligand Systeme, zur Tat-Protein-Flankierung, für die Bindung von Aptamer-Komplexen und zur Pegylierung.

Die Erfindung kann zur Behandlung von Virus-infizierten Zellen, Parasiten-infizierten Zellen, Bakterien-infizierten Zellen und Tumorzellen eingesetzt werden.

Die Erfindung soll nachstehend anhand von in der Zeichnung dargestellten Ausführungsbeispielen näher erläutert werden.

Es zeigen:

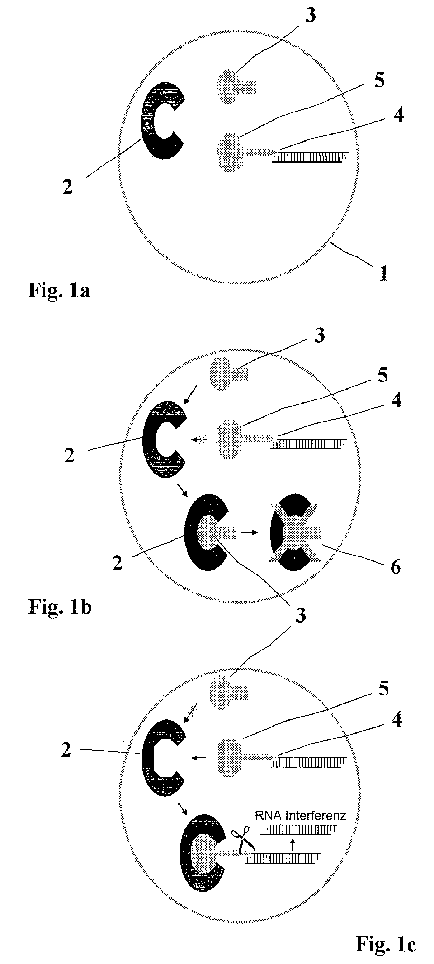

- Fig.1a-1c:: schematische Darstellung zur alternativen Beeinflussung von Zellen durch in diese eingebrachte biologisch wirksamen Moleküle, bestehend aus einem Proteaseinhibitor und einer Peptid-inhibierten siRNA.
- a):: Zelle mit zu hemmender spezifischer Zielprotease und den eingebrachten biologisch wirksamen Molekülen (Proteaseinhibitor und inaktive Peptid-inhibierte siRNA)
- b):: physiologische Beeinflussung der Zelle durch den Proteaseinhibitor
- c):: physiologische Beeinflussung der Zelle durch die aktivierte siRNA
- Fig. 2:: biologisch wirksame Moleküle mit kovalenter Bindung zwischen dem Proteaseinhibitor und der Peptid-inhibierten siRNA.
- Fig. 3:: biologisch wirksame Moleküle mit nicht kovalenter Bindung zwischen dem Proteaseinhibitor und der Peptid-inhibierten siRNA und Verabreichung in einem Komplex, beispielsweise einem Transfektionssystem

In **Fig. 1a** ist eine Virus-infizierte Zelle 1 mit einer in ihrer Wirkung zu hemmenden Zielprotease 2 dargestellt. In diese Virus-infizierte Zelle 1 wird erfindungsgemäß ein biologisch wirksames Molekül, bestehend aus einem an sich bekannten Proteaseinhibitor 3, beispielsweise Invirase, Norvir, Pentothal, Amprenavir oder Viracept für HIV-Infektionen, für die Zielprotease 2 und einer durch eine Peptidbindung 4 inaktiven ebenfalls an sich bekannten Peptid-inhibierten siRNA 5, gegeben. Die Peptidbindung 4 der Peptid-inhibierten siRNA 5 kann zum Zweck deren Aktivierung durch die Zielprotease 2 aufgebrochen werden, wofür die Proteinsequenz der Peptidbindung 4 an der Aufbruchstelle der exakten oder leicht veränderten Zielproteasensequenz entspricht.

**Fig. 1b** zeigt schematisch, wie der Proteaseinhibitor 3 bestimmungsgemäß an die Zielprotease 2 anbindet und diese wirksam hemmt. In diesem Fall wird die Peptidbindung 4 der Peptid-inhibierten siRNA 5 durch die Zielprotease 2 nicht aufgebrochen und die in ihrer schädigenden Wirkung inaktivierte Zielprotease 2 geht in eine bezüglich dieser Wirkung gehemmte Protease 6 über. Die Peptid-inhibierte siRNA 5 verbleibt wirkungslos in der Zelle 1.

Für den Fall, dass der Proteaseinhibitor 3 jedoch nicht oder nicht vollständig an die Zielprotease 2 anbinden und diese nicht bzw. in ihrer schädigenden Wirkung nicht gänzlich hemmen kann (beispielsweise durch leichte Mutation), behält die Zielprotease 2 zumindest eine gewisse Restaktivität, mit welcher sie in der Lage ist, die besagte Peptidbindung 4 der Peptid-inhibierten siRNA 5 aufzubrechen. In diesem Fall zeigt **Fig. 1c****,** wie nicht der Proteaseinhibitor 3, sondern die Peptid-inhibierte siRNA 5 an die Zielprotease 2 anbindet. Mit der symbolisch angedeuteten Darstellung des Aufbruches der Peptidbindung 4 kann durch die nun aktivierte siRNA beispielsweise die Expression eines für die Zelle 1 überlebenswichtigen Gens ausgeschaltet werden und die Zelle 1 stirbt ab.

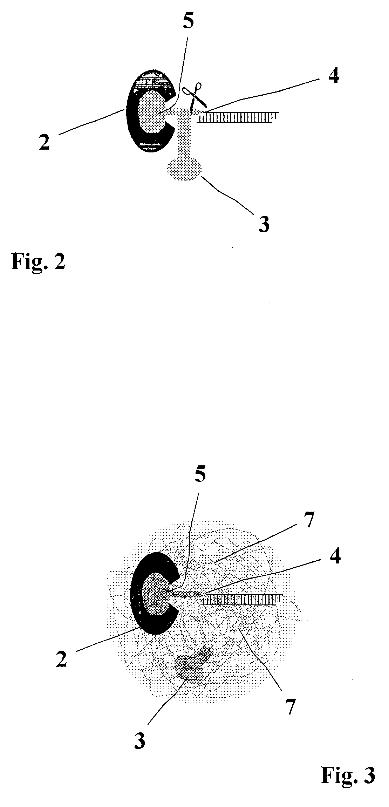

Die in die Zelle 1 zu deren physiologischen Beeinflussung einbringbaren biologisch wirksamen Moleküle können, insbesondere zum Zweck der gemeinsamen Verabreichung, eine kovalente Bindung zwischen dem Proteaseinhibitor 3 und der Peptid-inhibierten siRNA 5 mit der Peptidbindung 4 (vgl. **Fig. 2**) aufweisen. Dabei ist die Verbindung sterisch so gestaltet, dass der Proteaseinhibitor 3 an die Zielprotease 2 anbinden und diese inhibieren kann. Bei einer möglichen Unwirksamkeit des Proteaseinhibitors bzw. Wirksamkeit der nicht oder nicht vollständig inhibierten Zielprotease (vgl. **Fig. 1c**) wird das die siRNA 5 inhibierende Peptid zusammen mit dem Proteaseinhibitor 3 von der siRNA 5 zum Zwecke deren Aktivierung in beschriebener Weise abgespalten.

Die biologisch wirksamen Moleküle können auch in einem Komplex 7, beispielsweise aus Polyethylenimine, Dextran oder Polyethylenglycol (vgl. **Fig. 3**) angewandt werden. Dabei dient ein solcher Komplex insbesondere als nicht explizit dargestelltes Transfektionssystem zum Zwecke der Einbringung in Zellen und/oder zur Stabilisierung des Proteaseinhibitors 3 und der Peptid-inhibierten siRNA 5. Darüber hinaus sind in Fig. 3 der Proteaseinhibitor 3 und die Peptid-inhibierte siRNA 5 mit nicht kovalenter Bindung miteinander dargestellt. Eine nicht kovalente Bindung ermöglicht zudem auch eine getrennte und ggf. zeitlich versetzte Applikation der Molekülbestandteile.
Die Verwendung der biologisch wirksamen Moleküle zusammen mit einem besagten Komplex 7 kann jedoch auch unter kovalenter Bindung des Proteaseinhibitors 3 und der Peptid-inhibierten siRNA 5 erfolgen.

**Aufstellung der verwendeten Bezugszeichen**

| | |
|---|---|
| 1 | - Virus-infizierte Zelle |
| 2 | - Zielprotease mit schädigender Wirkung |
| 3 | - Proteaseinhibitor |
| 4 | - Peptidbindung |
| 5 | - inaktive Peptid-inhibierte siRNA |
| 6 | - gehemmte Protease |
| 7 | - Komplex (beispielsweise Transfektionssystem) |

## Patentansprüche

1. Biologisch wirksame Moleküle zur Beeinflussung von Virus-, Bakterien-, Parasiten-infizierten Zellen und/oder Tumorzellen, bestehend aus zumindest einem Proteaseinhibitor (3) für wenigstens eine spezifische Zielprotease (2) der Virus-, Bakterien-, Parasiten-infizierten Zellen und/oder Tumorzellen (1) sowie bestehend aus zumindest einer Peptid-inhibierten siRNA, PNA oder RNA (5), deren Peptidbindung (4) zum Zweck der Aktivierung der Peptid-inhibierten siRNA, PNA oder RNA (5) durch wenigstens eine spezifische Zielprotease (2) aufbrechbar ist.

2. Biologisch wirksame Moleküle gemäß Anspruch 1, **dadurch gekennzeichnet, dass** der zumindest eine Proteaseinhibitor (3) und die zumindest eine Peptid-inhibierten siRNA, PNA oder RNA (5) in einem Molekül vereint sind und in diesem kovalent miteinander verbunden sind.

3. Biologisch wirksame Moleküle gemäß Anspruch 1, **dadurch gekennzeichnet, dass** der zumindest eine Proteaseinhibitor (3) und die zumindest eine Peptid-inhibierten siRNA, PNA oder RNA (5), beispielsweise zum Zweck einer getrennten Applikation, nicht kovalent miteinander verbunden sind.

4. Biologisch wirksame Moleküle gemäß Anspruch 1, **dadurch gekennzeichnet, dass** die zumindest eine Peptid-inhibierten siRNA, PNA oder RNA (5) die exakte und/oder leicht veränderte Proteinsequenz der Aufbruchstelle der wenigstens einen spezifischen Zielprotease (2) enthält.

5. Biologisch wirksame Moleküle gemäß einem oder mehreren der Ansprüche 1 bis 3, **dadurch gekennzeichnet, dass** die Peptid-inhibierte siRNA, RNA oder PNA (5) wenigstens eine Anbindung weiterer Strukturen oder Funktionselemente besitzt, insbesondere Rezeptor-Ligand Systeme, Tat-Protein-Flankierung, Bindung von Aptamer-Komplexen und Pegylierung.

6. Zumindest ein Proteaseinhibitor zur Hemmung wenigstens einer spezifischen Zielprotease der Virus-, Bakterien-, Parasiten-infizierten Zellen und/oder Tumorzellen sowie zumindest eine Peptid-inhibierte siRNA, PNA oder RNA (5) mit gleicher und/oder leicht veränderter Proteinsequenz der Aufbruchstelle der wenigstens einen spezifischen Zielprotease zum Zweck der Aktivierung der Peptid-inhibierte siRNA, PNA oder RNA (5) durch die die Peptidbindung der Peptid-inhibierten siRNA, PNA oder RNA (5) aufbrechende wenigstens eine spezifische Zielprotease (2) für die Zellbeeinflussung von Virus-, Bakterien-, Parasiten-infizierten Zellen und/oder Tumorzellen zur Anwendung bei der Behandlung von Virus-, Bakterien-, Parasiten-infizierten Zellen und/oder Tumorzellen.

7. Verwendung von zumindest einem Proteaseinhibitor zur Hemmung wenigstens einer spezifischen Zielprotease der Virus-, Bakterien-, Parasiten-infizierten Zellen und/oder Tumorzellen sowie zumindest eine Peptid-inhibierte siRNA, PNA oder RNA (5) mit gleicher und/oder leicht veränderter Proteinsequenz der Aufbruchstelle der wenigstens einen spezifischen Zielprotease zum Zweck der Aktivierung der Peptid-inhibierte siRNA, PNA oder RNA (5) durch die die Peptidbindung der Peptid-inhibierten siRNA, PNA oder RNA (5) aufbrechende wenigstens eine spezifische Zielprotease (2) für die Zellbeeinflussung von Virus-, Bakterien-, Parasiten-infizierten Zellen und/oder Tumorzellen zur Herstellung eines Medikaments zur Behandlung von Virus-, Bakterien-, Parasiten-infizierten Zellen und/oder Tumorzellen.

8. Der zumindest eine Proteaseinhibitor sowie die zumindest eine Peptid-inhibierte siRNA, PNA oder RNA zur Anwendung gemäß Anspruch 6 oder Verwendung gemäß Anspruch 7, **dadurch gekennzeichnet, dass** der zumindest eine Proteaseinhibitor (3) und die zumindest eine Peptid-inhibierte siRNA, PNA oder RNA (5) gleichzeitig verabreicht werden.

9. Der zumindest eine Proteaseinhibitor sowie die zumindest eine Peptid-inhibierte siRNA, PNA oder RNA zur Anwendung gemäß Anspruch 6 oder Verwendung gemäß Anspruch 7, **dadurch gekennzeichnet, dass** der zumindest eine Proteaseinhibitor (3) und die zumindest eine Peptid-inhibierte siRNA, PNA oder RNA (5) getrennt und ggf. zeitlich versetzt verabreicht werden.

10. Der zumindest eine Proteaseinhibitor sowie die zumindest eine Peptid-inhibierte siRNA, PNA oder RNA zur Anwendung gemäß Anspruch 6 oder Verwendung gemäß Anspruch 7, **dadurch gekennzeichnet, dass** der zumindest eine Proteaseinhibitor (3) und die zumindest eine Peptid-inhibierte siRNA, PNA oder RNA (5) zusammen mit einem Transfektionsreagenz (7), insbesondere Lipide, Polyethylenimine, Dextran, Nanopartikel, Polymere, verabreicht werden.

11. Der zumindest eine Proteaseinhibitor sowie die zumindest eine Peptid-inhibierte siRNA, PNA oder RNA zur Anwendung gemäß Anspruch 6 oder Verwendung gemäß Anspruch 7, **dadurch gekennzeichnet, dass** gemeinsam mit der Verabreichung des zumindest einen Proteaseinhibitors (3) und der zumindest einen Peptid-inhibierten siRNA, PNA oder RNA (5) weitere Wirkstoffe verabreicht werden, die ebenfalls die zellulären Eigenschaften der Virus-, Bakterien-, Parasiten-infizierten Zellen und/oder Tumorzellen oder die Replikation, Infektiösität, Verkapselung oder Freisetzung des Virus, Replikation, Infektiösität, Stoffwechsel oder Freisetzung eines Bakteriums oder eines Parasiten beeinflussen.

## Claims

1. Biologically active molecules for influencing virus-, bacteria-, parasite-infected cells and/or tumour cells, consisting of at least one protease inhibitor (3) for at least one specific target protease (2) of the virus-, bacteria-, parasite-infected cells and/or tumour cells (1), and consisting of at least one peptide-inhibited siRNA, PNA or RNA (5), the peptide bond of which (4) can be broken up by at least one specific target protease (2) for the purpose of activation of the peptide-inhibited siRNA, PNA or RNA (5).

2. The biologically active molecules according to claim 1, **characterised in that** the at least one protease inhibitor (3) and the at least one peptide-inhibited siRNA, PNA or RNA (5) are combined in a molecule and are covalently bound to one another therein.

3. The biologically active molecules according to claim 1, **characterised in that** the at least one protease inhibitor (3) and the at least one peptide-inhibited siRNA, PNA or RNA (5) are not covalently bound to one another, for example for the purpose of separate application.

4. The biologically active molecules according to claim 1, **characterised in that** the at least one peptide-inhibited siRNA, PNA or RNA (5) contains the exact and/or slightly modified protein sequence of the breaking site of the at least one specific target protease (2).

5. The biologically active molecules according to one or more of claims 1 to 3, **characterised in that** the peptide-inhibited siRNA, PNA or RNA (5) has at least one linkage to further structures or functional elements, in particular receptor-ligand systems, tat protein flanking, bonds of aptamers complexes and pegylation.

6. At least one protease inhibitor for the inhibition of at least one specific target protease of the virus-, bacteria-, parasite-infected cells and/or tumour cells, and at least one peptide-inhibited siRNA, PNA or RNA (5) with the same or slightly modified protein sequence of the breaking site of the at least one specific target protease for the purpose of the activation of the peptide-inhibited siRNA, PNA or RNA (5) by means of the at least one specific target protease (2) breaking up the peptide bond of the peptide-inhibiting siRNA, PNA or RNA (5) for influencing the cells of virus-, bacteria-, parasite-infected cells and/or tumour cells for use in the treatment of virus-, bacteria-, parasite-infected cells and/or tumour cells.

7. Use of at least one protease inhibitor for the inhibition of at least one specific target protease of the virus-, bacteria-, parasite-infected cells and/or tumour cells, and of at least one peptide-inhibited siRNA, PNA or RNA (5) with the same and/or slightly modified protein sequence of the breaking site of the at least one specific target protease for the purpose of the activation of the peptide-inhibited siRNA, PNA or RNA (5) by means of the at least one specific target protease (2) breaking up the peptide bond of the peptide-inhibiting siRNA, PNA or RNA (5) for influencing the cells of virus-, bacteria-, parasite-infected cells and/or tumour cells for the preparation of a medicament for the treatment of virus-, bacteria-, parasite-infected cells and/or tumour cells.

8. The at least one protease inhibitor and the at least one peptide-inhibited siRNA, PNA or RNA for use according to claim 6 or the use according to claim 7, **characterised in that** the at least one protease inhibitor (3) and the at least one peptide-inhibited siRNA, PNA or RNA (5) are administered simultaneously.

9. The at least one protease inhibitor and the at least one peptide-inhibited siRNA, PNA or RNA for use according to claim 6 or the use according to claim 7, **characterised in that** the at least one protease inhibitor (3) and the at least one peptide-inhibited siRNA, PNA or RNA (5) are administered separately and, optionally, sequentially.

10. The at least one protease inhibitor and the at least one peptide-inhibited siRNA, PNA or RNA for use according to claim 6 or the use according to claim 7, **characterised in that** the at least one protease inhibitor (3) and the at least one peptide-inhibited siRNA, PNA or RNA (5) are administered together with a transfection reagent (7), in particular lipids, polyethyleneimines, dextran, nanoparticles, polymers.

11. The at least one protease inhibitor and the at least one peptide-inhibited siRNA, PNA or RNA for use according to claim 6 or the use according to claim 7, **characterised in that**, together with the administration of the at least one protease inhibitor (3) and the at least one peptide-inhibited siRNA, PNA or RNA (5), further active agents are administered which also influence the cellular properties of the virus-, bacteria-, parasite-infected cells and/or tumour cells or the replication, infectivity, encapsulation or release of the virus, replication, infectivity, metabolism or release of a bacterium or parasite.

## Revendications

1. Molécules à activité biologique, conçue pour influer sur des cellules infectées par des virus, bactéries ou parasites et/ou des cellules tumorales, formée d'au moins un inhibiteur de protéase (3) pour au moins une protéase cible (2) spécifique des cellules infectées par des virus, bactéries ou parasites et/ou des cellules tumorales (1), ainsi que d'au moins un pARNi (petit ARN interférent), ANP (acide nucléique peptidique) ou ARN (5), inhibé par un peptide et dont la liaison (4) à ce peptide peut, dans le but d'activer le pARNi, ANP ou ARN (5) inhibé par un peptide, être clivée par au moins une protéase cible (2) spécifique.

2. Molécules à activité biologique, conforme à la revendication 1, **caractérisée en ce que** l'inhibiteur de protéase (3), au nombre d'au moins un, et le pARNi, ANP ou ARN (5) inhibé par un peptide, au nombre d'au moins un, sont réunis en une seule molécule, au sein de laquelle ils sont liés l'un à l'autre par covalence.

3. Molécules à activité biologique, conforme à la revendication 1, **caractérisée en ce que** l'inhibiteur de protéase (3), au nombre d'au moins un, et le pARNi, ANP ou ARN (5) inhibé par un peptide, au nombre d'au moins un, par exemple en vue d'être utilisés séparément, ne sont pas liés l'un à l'autre par covalence.

4. Molécules à activité biologique, conforme à la revendication 1, **caractérisée en ce que** le pARNi, ANP ou ARN (5) inhibé par un peptide, au nombre d'au moins un, contient la séquence protéique exacte et/ou légèrement modifiée du site de clivage de la protéase cible (2) spécifique au nombre d'au moins une.

5. Molécules à activité biologique, conforme à l'une des revendications 1 à 3, **caractérisée en ce que** le pARNi, ANP ou ARN (5) inhibé par un peptide comporte au moins une liaison à d'autres structures ou éléments fonctionnels, en particulier système récepteur-ligand, éléments flanquant une protéine tat, liaison aptamère-complexe et pégylation.

6. Inhibiteur de protéase au nombre d'au moins un, conçu pour inhiber au moins une protéase cible spécifique de cellules infectées par des virus, bactéries ou parasites et/ou de cellules tumorales, et pARNi, ANP ou ARN (5) inhibé par un peptide, au nombre d'au moins un, comportant la séquence protéique, identique et/ou légèrement modifiée, du site de clivage de la protéase cible spécifique au nombre d'au moins une, dans le but d'activer le pARNi, ANP ou ARN (5) inhibé par un peptide au moyen de la protéase cible (2) spécifique au nombre d'au moins une qui clive la liaison au peptide du pARNi, ANP ou ARN (5) inhibé par un peptide, pour influer sur des cellules infectées par des virus, bactéries ou parasites et/ou des cellules tumorales, pour utilisation dans le traitement de cellules infectées par des virus, bactéries ou parasites et/ou de cellules tumorales.

7. Utilisation d'au moins un inhibiteur de, protéase, conçu pour inhiber au moins une protéase cible spécifique de cellules infectées par des virus, bactéries ou parasites et/ou de cellules tumorales, et d'au moins un pARNi, ANP ou ARN (5) inhibé par un peptide, comportant la séquence protéique, identique et/ou légèrement modifiée, du site de clivage de la protéase cible spécifique, au nombre d'au moins une, dans le but d'activer le pARNi, ANP ou ARN (5) inhibé par un peptide au moyen de la protéase cible (2) spécifique au nombre d'au moins une qui clive la liaison au peptide du pARNi, ANP ou ARN (5) inhibé par un peptide, pour influer sur des cellules infectées par des virus, bactéries ou parasites et/ou des cellules tumorales, pour la fabrication d'un médicament destiné au traitement de cellules infectées par des virus, bactéries ou parasites et/ou de cellules tumorales.

8. Inhibiteur de protéase au nombre d'au moins un, et pARNi, ANP ou ARN (5) inhibé par un peptide, au nombre d'au moins un, pour utilisation, conformes à la revendication 6, ou utilisation conforme à la revendication 7, caractérisés, ou **caractérisée, en ce que** l'inhibiteur de protéase (3) au nombre d'au moins un et le pARNi, ANP ou ARN (5) inhibé par un peptide, au nombre d'au moins un, sont administrés en même temps.

9. Inhibiteur de protéase au nombre d'au moins un, et pARNi, ANP ou ARN (5) inhibé par un peptide, au nombre d'au moins un, pour utilisation, conformes à la revendication 6, ou utilisation conforme à la revendication 7, caractérisés, ou **caractérisée, en ce que** l'inhibiteur de protéase (3) au nombre d'au moins un et le pARNi, ANP ou ARN (5) inhibé par un peptide, au nombre d'au moins un, sont administrés séparément, et le cas échéant, avec un décalage dans le temps.

10. Inhibiteur de protéase au nombre d'au moins un, et pARNi, ANP ou ARN (5) inhibé par un peptide, au nombre d'au moins un, pour utilisation, conformes à la revendication 6, ou utilisation conforme à la revendication 7, caractérisés, ou **caractérisée, en ce que** l'inhibiteur de protéase (3) au nombre d'au moins un et le pARNi, ANP ou ARN (5) inhibé par un peptide, au nombre d'au moins un, sont administrés conjointement avec un réactif de transfection (7), en particulier des lipides, des polyéthylène-imines, un dextrane, des nanoparticules, ou des polymères.

11. Inhibiteur de protéase au nombre d'au moins un, et pARNi, ANP ou ARN (5) inhibé par un peptide, au nombre d'au moins un, pour utilisation, conformes à la revendication 6, ou utilisation conforme à la revendication 7, caractérisés, ou **caractérisée, en ce que**, conjointement avec l'inhibiteur de protéase (3) au nombre d'au moins un et le pARNi, ANP ou ARN (5) inhibé par un peptide, au nombre d'au moins un, sont administrées d'autres substances actives qui influent aussi sur les propriétés cellulaires de cellules infectées par des virus, bactéries ou parasites et/ou de cellules tumorales, ou sur la réplication, l'infectiosité, l'encapsulation ou la libération d'un virus, ou sur la réplication, l'infectiosité, le métabolisme ou la libération d'une bactérie ou d'un parasite.
